# EUROPEAN PATENT APPLICATION

(11) **EP 2 008 716 A1**
(43) Date of publication of application: **31.12.2008**
(21) Application number: 07252628.8
(22) Date of filing: 28.06.2007
(51) Int. Cl.: B01L 3/00, G01N 33/28, G01N 21/01, G01N 21/03, G01N 30/60

(54) **Sample plate**

(71) Applicant: BP OIL INTERNATIONAL LIMITED, Sunbury-on-Thames, Middlesex TW16 7BP (GB)
(72) Inventor: The designation of the inventor has not yet been filed
(74) Representative: De Kezel, Eric

(57) **Abstract**

A sample plate, and portable apparatus comprising a sample plate and a base portion, which sample plate comprises a sample inlet, a reaction zone, an analysis zone, and at least one separation zone, the sample plate being adapted to allow;
(a) a sample fluid to be fed to the sample plate through the inlet to the reaction zone or optionally to a separation zone, which separation zone separates the sample fluid into two or more fractions at least one of which is fed to the reaction zone;
(b) a reactant to be fed to the reaction zone;
(c) the reaction zone to be maintained under conditions that enable reaction to occur between the reactant and the sample fluid or fraction thereof to produce a product fluid; and
(d) transfer of the product fluid to the analysis zone or optionally to a separation zone in which the product fluid is separated into two or more fractions, at least one of which is transferred to the analysis zone.

## Description

This invention relates to analytical apparatus, more specifically to a portable apparatus and method for determining the heteroatom concentration in a hydrocarbon fluid, for example a sample of crude oil or a crude oil derivative.

There are numerous sources of crude oil, with widely varying chemical and physical properties. For example, crude oils such as West Texas Intermediate have a high proportion of relatively low-boiling hydrocarbon components, whereas Venezuelan crude oils for example tend to comprise a higher proportion of higher-boiling components. Due to the wide variation in crude oil types, it is advantageous for an oil refinery to be able to use as wide a variety of crude oil feedstocks as possible in order to reduce reliance on any single crude oil source, and also to allow opportunities for low cost feedstocks to be used when available.

Different crude oils, as a result of their different compositions, often behave very differently within the refinery and hence their impact on refining operations and process equipment will also vary. Therefore, analysis of a crude oil that has been procured or is to be procured is important in establishing its suitability for use, and the likely extent of any impact on the refinery processes and/or equipment. Descriptions of refinery processes, and products derived therefrom, are well-known to the person skilled in the art, and are described, for example, under the chapter entitled "Oil Refining", by Walther W. Irion and Otto S. Neuwirth, in Ullmann's Encyclopedia of Industrial Chemistry, published by Wiley.

It is not uncommon for feedstocks to be purchased while crude oil is still in transit, for example in a crude oil tanker. Without proper analytical data, a purchaser of a crude oil cargo must make a number of assumptions on the value of the feedstock in order to determine whether or not to make a trade. It would be advantageous if analytical data of a cargo could be made rapidly, so that the results can be made available to the potential purchaser in a timely manner.

The rapid analysis of products of refinery processes is also desirable. Such products include intermediates in the overall refinery process, bitumen, products from the overall refinery process which are subsequently used as chemical feedstocks and products from the overall refinery process which are subsequently used as fuels or lubricants, or as blending components for fuels or lubricants, as well as the fuels (e.g. aviation fuel, gasoline, diesel and marine fuels) and lubricants themselves. The rapid analysis of formulated products, fuels and lubricants is also desirable, for example at a terminal, at a pipeline, in the distribution system, or at a point of sale.

Analysis of crude oil for properties such as boiling point range, acidity/basicity, asphaltenes content, heteroatom content for example nitrogen and/or sulphur content, and aromatics content. This can be achieved by transporting a sample of crude oil to a laboratory equipped with appropriate analytical facilities. This often requires a large quantity of sample, and can be time consuming. Known techniques for the analysis of nitrogen or sulphur-containing compounds, for example, include those described in ASTM D1552. On-line measurements can be made, where suitable on-line analysers are available, and where it is possible to make such measurements. Known on-line analytical techniques include on-line gas chromatography and on-line optical absorption and/or spectroscopic techniques. However, on-line apparatus is typically installed on the relevant parts of processing plant at manufacturing facilities, and is therefore not transportable to remote locations.

According to the present invention, there is provided a sample plate for a portable analysis apparatus for the analysis of a sample fluid, which sample plate comprises a sample inlet, a reaction zone, an analysis zone, and at least one separation zone, the sample plate being adapted to allow;
(a) a sample fluid to be fed to the sample plate through the inlet to the reaction zone or optionally to a separation zone, which separation zone separates the sample fluid into two or more fractions at least one of which is fed to the reaction zone;
(b) a reactant to be fed to the reaction zone;
(c) the reaction zone to be maintained under conditions that enable reaction to occur between the reactant and the sample fluid or fraction thereof to produce a product fluid;
(d) transfer of the product fluid to the analysis zone or optionally to a separation zone in which the product fluid is separated into two or more fractions, at least one of which is transferred to the analysis zone.

According to a second aspect of the present invention, there is provided use of the sample plate in the analysis of a product fluid resulting from reaction between a reactant and a fluid sample or fraction thereof.

The sample plate of the present invention can be combined with a base portion to form a portable analysis device. The portable analysis device is designed to be readily transportable so that analysis is not restricted to laboratory or manufacturing site locations. For example, it can be easily transported to and used for the analysis of the fluid contents of a remote storage tank, a ship cargo, a rail wagon or a road tanker, for example.

A sample fluid is added to the sample plate through the sample inlet, wherein it is directed either to the reaction zone, or optionally to a separation zone disposed between the inlet and the reaction zone, in which the sample fluid can be separated into two or more fractions, at least one of which can be fed to the reaction zone. Fluid communication means between various portions of the sample plate are typically in the form of micro-fluidic channels.

The sample plate also comprises a means for transferring a reactant to the reaction zone. In one embodiment, the reactant is contacted with the sample fluid or fraction thereof before being fed to the reaction zone. Alternatively, the sample fluid (or fraction thereof) and reactant can be separately fed into the reaction zone.

The reaction zone is adapted to allow reaction to occur, for example by being held at elevated temperature and/or by ensuring thorough mixing of the reactant and fluid sample. In one embodiment, the reaction zone comprises a means of agitation to ensure efficient mixing of the reactant and fluid sample or fraction thereof. For example through the use of a stirrer such as a magnetic stirrer, control of which can be provided by suitable actuating devices located in the base portion of the apparatus. In an alternative embodiment, the reaction zone is adapted to ensure sufficient turbulence is created when the reactant and fluid sample or fraction thereof are fed thereto. In a further embodiment, the reactant and fluid sample are fed counter-currently. The exact dimensions of the reaction zone, and any fluidic channels associated therewith, depend inter alia on the nature of the fluid sample, the reactant, and their feed velocity into the reaction zone.

The reaction between the fluid sample, or fraction thereof, and the reactant produces a product stream. The product stream can be fed to the analysis zone directly. Alternatively, it can be fed to a separation zone in which the product stream is separated into two of more fractions, at least one of which is fed to the analysis zone.

The sample plate comprises at least one separation zone. Optionally, there can be more than one separation zone, for example two separation zones in which one is situated upstream and one downstream of the reaction zone. A separation upstream of the reaction zone is typically used to ensure that only a desired fraction of the fluid sample is analysed. Separation downstream of the reactor is typically employed to ensure that only a specified fraction of interest is analysed.

Separation can be achieved by means of a micro-fluidic fractionation or micro-distillation device for fractionation of the fluid according to boiling point of constituent components. In an alternative embodiment, separation can be achieved by feeding the sample fluid (or fraction thereof) or the product stream over a solid-phase separation medium in the presence of a carrier gas or liquid, which enables separation into two or more fractions to be achieved, based on characteristics such as polarity. The composition of the fractions will depend on the surface characteristics of the solid-phase separation medium (often referred to as the stationary phase). The separated components can then be independently analysed, for example in either the same analysis zone (such as the optical analysis zone), or in different analysis zones.

Where used, a micro-distillation device may be a micro engineered device comprising a micro-heater for vaporising the fluid sample, a suitable channel, for example a capillary, through which the vaporised sample passes, or a series of channels such that vapour liquid exchange is achieved (such as in a counter current device), a suitable condensing zone (typically a cooled zone, such as a micro-refrigerator) on which vaporised sample that has passed up the channel condenses, optionally fitted with a micro-sensor to measure the condensation of sample at the condensing zone, which in one embodiment can be an optical sensor. The micro-distillation device can be a micro-fabricated separation device, for example on a silicon wafer.

Where separation using a solid stationary phase is used, the separation zone can be in the form of a chamber or channel containing a solid stationary phase with the desired surface characteristics. A solvent is typically used to assist separation of the fluid (for example the sample fluid or product stream) into the desired fractions. In one embodiment, the polarity of the solvent can be varied with time in order to improve separation. This can be achieved, for example, by mixing a relatively non-polar with a relatively more polar fluid in order to change polarity with time. The shape and dimensions of the chamber, the quantity of solid and the shape and size of the solid particles, and the choice of solvent is selected to ensure a balance is maintained between separation that is efficient to ensure accuracy of results, yet also sufficiently rapid so as to allow a quick analysis.

The sample plate of the present invention is particularly suited for the analysis of a complex fluid sample. A complex fluid is one that comprises a plurality of different components. Examples of complex fluids include those comprising a plurality of hydrocarbons, such as crude oil, process streams and product streams produced by a crude oil refinery, and product streams derived from Fischer-Tropsch synthesis processes.

The analysis zone receives the product stream, or a fraction separated therefrom. The analysis zone is adapted either with one or more sensors for performing the desired analysis, or is adapted to allow one or more sensors located elsewhere, for example in the base portion, to perform the appropriate measurements.

Examples of analysis devices that can be associated with the portable apparatus include micro conductivity/capacitance devices (e.g. for measuring acidity), micro rheological devices (e.g. for viscosity), micro mass spectrometer, a micro oscillator device (e.g. for measuring boiling point profile), micro-GC or micro-LC devices (e.g. for separating and analysing gas-phase or liquid-phase components), and optical analysis devices suitable, for example, in the measurement of near infrared (NIR), ion mobility/differential mobility, acousto-optical, acoustic, UV-visible and mid infrared (MIR) wavelengths. Optionally, the portable apparatus is adapted to perform more than one analysis, for example having more than one analysis device associated with more than one analysis zone on the sample plate.

Suitable micro-oscillators are described in US 5,661,233 and US 5,827,952. Suitable optical analysis devices for measuring NIR spectra include the Axsun NIR-APS Analyser produced by Axsun Technologies Inc., Massachusetts. Suitable micro-GC devices include Siemens MicroSAM process GC's or SLS Micro-technology GC's. Suitable micro- ion mobility/differential mobility spectrometers include the Sionex microDMx.

Where suitable micro-devices are not available, the portable apparatus may be used in combination with other portable analysers, particularly those yielding elemental data, such as portable X-Ray Fluorescence (XRF) spectroscopy and Laser Induced Breakdown Spectroscopy (LIBS) to improve the number of techniques available for analysing the liquid sample. XRF, for example, can provide analysis of sulphur and metals content of a sample, for example of crude oil fractions. Suitable, portable, XRF analysers include those available from OXFORD instruments

In one embodiment, the analysis zone of the sample plate is suitable for performing optical analysis, in which it can allow one or more wavelengths of electromagnetic radiation (EMR) to be directed therein and also to allow EMR transmitted through and/or reflected by the sample to be emitted from the analysis zone. This can be achieved through the use of windows that are suitably transparent to the EMR being used. For example, glass or quartz windows are suitable where visible EMR wavelengths are used.

Where the analysis zone is for optical analysis, the portable analysis apparatus comprises an optical analysis device, with an associated sensor which is capable of converting electromagnetic radiation (EMR) transmitted through and/or reflected by the contents of the analysis zone of the sample plate into electronic signals. The optical analysis device also comprises an EMR source. Adsorption of EMR by the contents of the optical analysis zone can be measured at a particular wavelength or over a range of wave lengths in order to monitor specific compounds, a range of compounds, or a specific type of molecular species.

In a preferred embodiment of the invention, the sample plate is replaceable, in that it can be removed from the base portion after one analysis, and replaced with another sample plate, thus avoiding contamination between samples. The replaced sample plate can then either be cleaned for subsequent re-use, or even disposed of if contamination risk cannot be circumvented by cleaning. Attachment means can be provided provided, such as click-fit type connectors or slot in type connectors, using guide rails for example.

Parts of the portable apparatus that come into direct contact with sample or other fluids such as precipitant or solvent are preferably located on the sample plate, while devices that are complex, expensive or difficult to fabricate or replace are preferably located within the base portion of the portable apparatus. Thus, analytical devices such as EMR sensors, EMR sources, actuating mechanisms for pumps and valves, microprocessors for controlling the sequence of actuation and analysis, and so on are preferably located in the base portion of the portable apparatus. This ensures that reusable parts of the equipment do not suffer damage through contamination or through cleaning, for example, or are not lost through disposal of the sample plate, thus minimising costs.

The portable apparatus is typically provided with suitable pumping and measuring means, and also valves to appropriately direct the various fluids to the appropriate regions of the sample plate when in use. Associated driving and actuating apparatus is typically located in the base portion. Suitable micro-pumps include diaphragm pumps, peristaltic pumps, rotary pumps, gear pumps and piston pumps. Additionally, an air pump, taking air from the surrounding atmosphere, can be used to transfer liquids around the plate, and/or to flush relevant transfer lines or zones such as the reaction and analysis zones. Pumps can be adapted to measure the volumes of fluids so that accurate quantitative information can be obtained.

The portable apparatus is suitably adapted so that processing of the relevant analytical and volumetric information can be achieved, and results in the form of an electronic or printed output can be provided. This suitably achieved with a microprocessor that can run suitable programs to ensure the correct volumes of fluids are pumped around the sample plate, and in the correct sequence. In one embodiment, the micro-processor is programmable so that existing analysis, pumping and sequence programmes can be improved, or different analyses for different samples can be performed.

In one embodiment, the portable apparatus can be adapted so that different portions of the sample plate can be heated or cooled using suitable heating or cooling devices. These can also be linked in with the processor so that they can be turned on or off as necessary. Thus, for example, the reaction zone can be adapted to accommodate heating elements located in the base portion, and which allow the temperature in the reaction zone to be raised to a required reaction temperature. The one or more separation zones can also be adapted, for example to provide a temperature ramp to allow sequential removal of the various fractions of the sample fluid and/or the product fluid within the separation zone, or to control the temperature of a micro-distillation device. The temperature control means can be located on the sample plate, for example by having resistively heated elements or wires associated with the region or zone of the sample plate to be heated, and which interface with an electrical power source located in the base portion of the portable apparatus for example. Alternatively, the sample plate can be adapted to accommodate temperature control means which are located within the base portion, such that the desired temperature control can be achieved.

The portable apparatus can comprise, or can at least be compatible with, wireless communications, such as a wireless mesh network, and also with remote communications means, such as satellite-based data communication, such that the analysis results may be readily communicated to the potential purchaser, again reducing the time-scale on which the analysis data is available to the potential purchaser.

The use of a portable apparatus according to the present invention requires only a small quantity of liquid sample, typically less than 20 ml, preferably 10 ml or less.

The portable apparatus can be adapted to store liquid sample, reactant, and optionally any other reagents that are used in the analysis of the liquid sample, for example a solvent for facilitating mixing of liquid sample with the reactant, a catalyst used for improving the reaction rate, or any other reagents for further analysis or sample treatment that may be carried out on the sample plate.

Storing fluid sample, reactant and/or any other fluid reagents on the sample plate helps to minimise the quantity of materials, in that the sample plate can be provided with a quantity sufficient to perform the desired analysis, and prevents the necessity for transporting a number of reagent vessels when analysis remote from a laboratory is required. In one embodiment, different sample plates can be provided with different liquids which may be needed for analysing different samples. Thus, a single portable apparatus can be provided with a plurality of different sample plates that are adapted to perform different analysis, but which can be attached to and be used with a single portable apparatus. This allows different analyses to be performed successively using a single portable apparatus but different sample plates. In this way, only the specific fluids or reagents required for a particular analysis, and in the amount needed for that analysis, needs to be stored.

Where the sample plate is not adapted to store reagents, then the sample plate is adapted to receive the reagents from external sources of reagent, for example located in the base portion of the apparatus or in reagent bottles or other storage means, such as a reagent-filled syringe, optionally associated with micro-pumps for pumping and measuring volumes of reagents that are fed to the sample plate. In one embodiment, the reagent reservoir can be the barrel of a syringe that, together with a plunger, acts as a piston pump.

Suitable materials from which the sample plate can be made include ceramic, metal, glass, polymeric materials, or a combination of two or more thereof. Polymeric materials which can be injection moulded are particularly suitable. Preferably, the sample plate is resistant to leaching or other degradative effects caused by the action of the sample fluid, solvent, precipitant, or any other fluids used in the analysis. For the analysis of crude oil or products of refinery processes, particularly suitable polymeric materials include polyetheretherketone (PEEK), polyphenylene sulphide (PPS) and polytetrafluoroethylene (PTFE), which show good resistance to degradation and leaching in the presence of crude oil and other hydrocarbon-based products.

The various components and devices of the portable apparatus, are suitably microfabricated, in which techniques such as micro-moulding, electrodischarge machining, or laser micro-machining are employed in their fabrication, for example in cutting or etching microfluidic channels in the sample plate, and in the manufacture of microvalves or micropumps. Microfabricated components also include those manufactured by techniques employed in the micro-chip industry, for example sensors and microprocessors.

A sensor is typically used to produce an electrical signal in response to a stimulus, such as contact with a sample fluid, the solution of precipitate, or when exposed to EMR. This electrical signal is fed to an associated set of electronics which converts the input signal into a value for the property being measured.

Because of the relatively small size of the components of the portable analysis apparatus of the present invention, the power requirements are also relatively low. Hence, the portable analysis apparatus may be operated from a suitable battery (or battery pack), preferably a rechargeable battery, without the battery requirements being too heavy to impact the portability of the apparatus.

The sample plate and portable apparatus of the present invention can be advantageously used in the analysis of a complex fluid, for example fluids associated with the petroleum or petrochemical industries, for example in the fields of oil exploration, production, refining or petrochemicals production. Thus hydrocarbon fluids, exploration fluids, refinery feedstocks, refinery intermediates, products of refining such as fuels or lubricants, fluids used as treatments for or additives to such fluids.

Preferably the refinery feedstock is a crude oil or blend of crude oils, optionally also comprising or being blended with one or more of a synthetic crude component, a biocomponent or an intermediate component, such as a residue component or a cracked stock component.

The reactant can comprise a single compound or more than one compound. A catalyst may also be fed to, or may be present within, the reaction zone which can reduce reaction time between the reactant and sample fluid or fraction thereof, resulting in a reduction in the total analysis time. A solvent can be added in order to facilitate mixing between the components fed to the reaction zone. This is typically achieved by dissolving all the separate fluids to create a single liquid phase, or by enabling emulsification to improve mixing between immiscible fluids.

In a further embodiment, a phase transfer catalyst or surfactant can be used together with or instead of a solvent. Examples of compounds that can act as phase transfer catalysts or surfactants include mono, di or tri alkyl ammonium or alkyl phosphonium salts, in particular those having at least one long hydrocarbon chain, for example having 6 or more carbon atoms, preferably 10 or more carbon atoms. In one embodiment of the process of the present invention, the sample fluid is a crude oil-derived hydrocarbon stream, the oxidant is aqueous hydrogen peroxide, and the acid is phosphotungstic acid. Addition of dioctadecyl dimethyl ammonium chloride helped to stabilise the emulsion. This is thought to be due to the formation of an dioctadecyl dimethyl ammonium salt of phosphotungstic acid, such that the organic octadecyl chain imparts greater solubility in the hydrocarbon-based sample fluid, while the phosphotungstate anion remains soluble in the aqueous portion of the mixture.

In one embodiment of the invention, the portable apparatus is used for determining the concentration of nitrogen and/or sulphur containing compounds in a fluid sample, such as a sample of crude oil or a process stream from a crude oil refinery. In the case of sulphur, there are many types of sulphur-containing compounds associated with crude oil, and determining the total sulphur content is typically a complicated process, requiring separate analysis of several known compounds, involving detailed and time-consuming calibration and analysis. Therefore, in the process of the present invention, the sulphur-containing compounds are converted into a single sulphur-containing compound or into a single class of sulphur-containing compounds.

As an example, in the production of a diesel fuel in a crude oil refinery, thiophenic compounds present in the crude oil tend to remain present in process streams that are used to produce diesel fuel, even after purification or polishing processes such as hydrocracking and hydrotreating. It is therefore important to know the total concentration of such sulphur-containing compounds in the final diesel fuel in order to establish whether the fuel meets the relevant specifications.

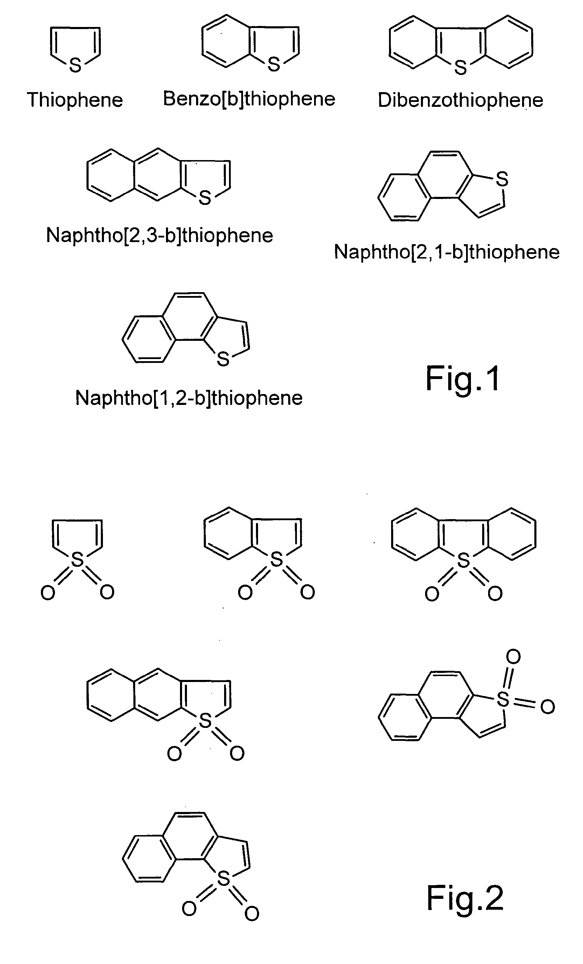

Examples of thiophenic compounds that may be present in refinery streams and diesel fuel are shown in Figure 1, and include thiophene, benzo-[b]-thiophene, dibenzothiophene, naphtho-[2,3-b]-thiophene, naphtho-[2,1-b]-thiophene and naphtho-[1,2-b]-thiophene. Such compounds can be oxidised to the corresponding sulphones, comprising S=O bonds, which can be readily detected and quantified using optical techniques, for example UV-visible, infrared or near infrared absorption techniques. The sulphones corresponding to the thiophenic compounds of Figure 1 are shown in Figure 2.

Thus, in one embodiment of the present invention, the reactant is an oxidant and the fluid sample is crude oil or a composition resulting from a crude oil refinery process, which comprises one or more sulphur-containing compounds, such as one or more thiophenic compounds, than can be oxidised to corresponding sulphones. In a further embodiment, the sample fluid is a diesel fuel, either before or after being hydrotreated.

The reactant should be capable of efficiently and rapidly causing the oxidation, in order to reduce the overall time taken for the reaction and analysis. Peracids, peroxides and hydroperoxides are examples of oxidants that can be used, as are peroxy salts of inorganic ions, for example persulphate, perchlorate, perbromate or periodate. Further examples of oxidising agents that can be used include selenium dioxide, sulphuric acid, ozone, RuO₄ and OsO₄. Hydrogen peroxide has been found to be a particularly efficient and rapidly acting oxidant for the conversion of thiophenic sulphur moieties to corresponding sulphones, particularly when used in combination with a solvent in order to improve mixing between the aqueous peroxide and hydrophobic fluid samples, such as crude oil or compositions resulting from the refining thereof. The solvent can be selected from one or more of toluene, methanol, dichloromethane and heptane.

A catalyst can optionally be employed in order to improve the rate of reaction between the reactant and the liquid sample. Thus, where hydrogen peroxide is used as reactant for a liquid sample of a diesel fuel or precursor thereof, an acid can be employed to act as a catalyst to increase the oxidation rate of thiophenes to the corresponding sulphones. Acids that are particularly suitable for use include one or more of sulphuric acid and heteropolyacids such as silicotungstic acid and phosphotungstic acid.

Fluids fed to the reaction zone can be fed separately thereto. Alternatively, two or more fluids can be pre-mixed before being fed to the reaction zone.

Because analysis obtainable from the portable apparatus of the present invention is rapid, analyses can be obtained more often and/or can be used for process optimisation. For example, the portable analysis apparatus may be used at a refinery and regular analyses can be performed on blends of refinery feedstocks, such as blends of crude oils, produced (from two or more sources available) at the refinery, so as to enable the refinery configuration to be optimised for the blend. Further the portable analysis apparatus may be used to verify consistency and/or quality of feedstocks on arrival at a refinery or blending station and/or may be used to provide on-line or at-line determination of feedstock quality and property data for input to blending and process refinery optimisation models.

Where the portable apparatus of the present invention is used for analysis of crude oil, for example at the "well-head" on a drilling site, a number of apparatus may be operated at different well-heads which use a common transport mechanism, for example a common pipeline, to provide analysis of the crude oil from each well. Analysis of the individual crude oils and appropriate scheduling may allow more optimum composition of the final crude oil blend. In addition, by repeated analysis of the crude oils from different well-heads, changes in the individual crude oils with time can be used to predict the effects on the produced crude oil blend, or influence the blending to maintain a constant quality crude oil blend.

Similarly, where the portable analysis apparatus is used for analysis of a product derived from a refinery process, it may be used to check consistency and quality of the product at the refinery, or at subsequent locations, such as at chemical plants themselves, at fuels blending terminals or in fuel-containing tanks, such as in fuel tankers or stationary tanks at airports, dockyards or on petrol station forecourts.

The invention is further illustrated by the accompanying non-limiting example and the Figures, in which:
Figure 1 shows structural formulae of examples of thiophenic species that can be found in crude oil or refinery process streams derived therefrom.
Figure 2 shows structural formulae of sulphones corresponding to the thiophenic species of Figure 1.
Figure 3 is an exploded schematic illustration of a sample plate for the determination of thiophenic sulphur in a crude oil-derived sample fluid.
Figure 4 is an overhead view of a sample plate according to the present invention, in which channels relating to the introduction of reactant, solvent and sample fluid, the separation zones, and the reaction zone are highlighted.
Figure 5 is an overhead view of the same sample plate as shown in figure 4, in which the waste channels are highlighted.
Figure 6, 7, 8 and 9 illustrate the positions of the microvalves of the sample plate of Figures 4 and 5 at various stages of an analysis cycle.
Figure 10 illustrates a UV/Visible analysis cell for use with a sample plate and corresponding base portion of a portable apparatus according to the present invention.
Figure 11 illustrates an alternative UV/Visible analysis cell for use with a sample plate and corresponding base portion of a portable apparatus according to the present invention.

Figure 1 shows examples of the types of thiophenic sulphur compounds that can be found in crude oil refinery process streams, and which can end up in diesel fuel derived therefrom. Figure 2 shows the corresponding sulphones which are produced as a result of an oxidation reaction as hitherto described, the S=O of which are strongly absorbing in the UV/Visible region of the spectrum.

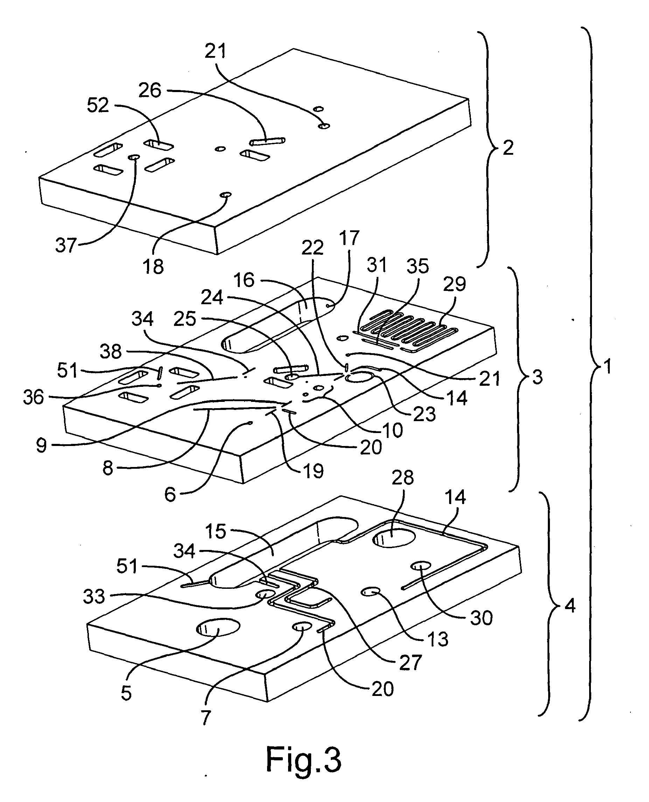

Figure 3 shows an exploded view of a sample plate 1 according to the present invention, and which can be used in the determination of thiophenic sulphur in a hydrocarbon sample, such as a crude oil sample, a composition resulting from refining crude oil, or a pre- or post-hydrotreated diesel fuel. The sample plate is fabricated from three sub-plates, 2, 3 and 4.

Sub-plate 2 in general comprises the inlet for the sample injection (diesel in this case) 18, the inlet for reactant 21, together with vent 37 from the concentration zone 36 (identified on sub-plate 3), and which also acts as an insertion point for a syringe used for extracting sample from the concentration zone. The sample plate comprises two groups of holes which accommodate heating elements from the base portion of the apparatus, the first of which 26 surrounds the reaction zone 25, and the second of which 52 surrounds the concentration zone 36.

Sub-plate 3 in general comprises the microfluidic transfer channels 8, 9, 14, 19, 20, 22, 23, 24, 29, 31, 34, 35, 38 and 51, separation zone 10 comprising two separation columns, separation zone 32 comprising a single separation column, reaction zone 25, the charcoal filter bed 16, the vent 17 for the charcoal filter bed, and the opening to concentration zone 36, the main part of which is within the body of the sub-plate 3, and is not seen in this view. Sub-plate 3 also comprises the remainder of the reactant inlet 21, which leads to microfluidic channel 22, and heptane inlet 6, which leads to microfluidic channel 19.

Sub-plate 4 generally comprises the waste reservoir 15, the microfluidic channels leading thereto 14, 20, 27, 34 and 51, and the reservoirs for heptane (a solvent) 5, and a mixture of heptane/1% methanol (a second solvent) 28.. The sub-plate also comprises recesses that house microvalves 7, 13, 30, 33.

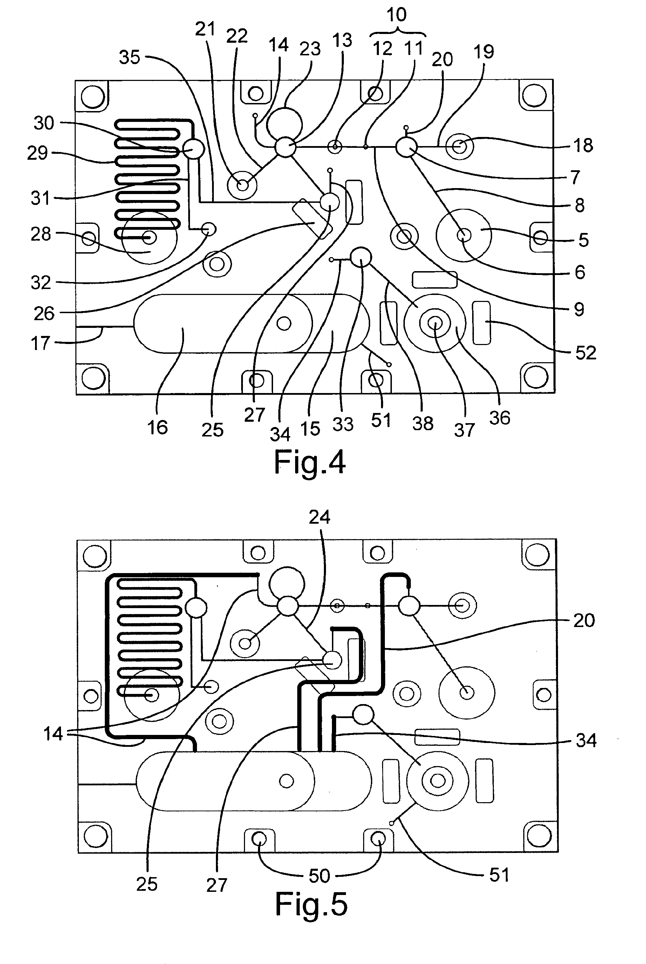

Figure 4 shows an overhead view of the same sample plate illustrated in Figure 3. Highlighted are the channels relating to the flow of sample, solvent and reactant through the inlets, the reaction zones and the separation zones, and which are present in sub-plate 3. Figure 5 is a view of the same sample plate as Figure 4, in which channels in sub-plate 4 leading to the waste reservoir 15 are highlighted.

The following provides a description of how the sample plate is used in the analysis of thiophenic sulphur in a diesel fuel sample.

The sample plate comprises a first 10 and a second 32 separation zone, one of which is located upstream of the reaction zone 25 and the other of which is located downstream of the reaction zone.

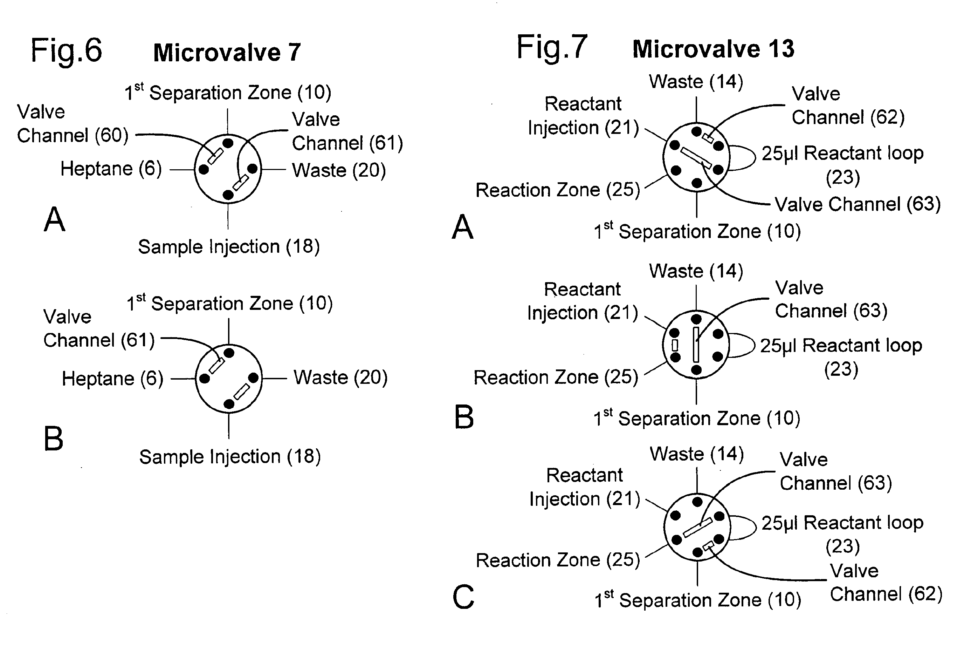

The reactor is pre-loaded with 10mg of [((CH₃)₂(C₁₈H₃₇)₂)N]₃[PW₁₂O₄₀] which is to act as a phase-transfer catalyst. Valve 13 is positioned such that a 30% H₂O₂ solution (the reactant, in this case an oxidant) can be fed from a reservoir (not shown) through reactant inlet 21, through microfluidic channel 22 and microvalve 13 to fill reactant loop 23, designed to hold 25 µl of the oxidant solution, and then into waste channel 14. When the loop is filled, the valve is then rotated, which seals the 25ul loop of hydrogen peroxide solution, and allows passage of any fluid leaving separation zone 10 into microfluidic channel 14, which in turn leads to the waste reservoir 15. The microvalve 13 comprises two valve channels (62 and 63, shown in Figure 7), which connect the different microfluidic channels, depending on the valve position, discussed further below in relation to Figure 7.

Heptane solvent is then pumped from a heptane reservoir 5 through heptane inlet 6 to microvalve 7 through microfluidic channel 8. Microvalve 7 is set at this point to allow the heptane to pass through to microfluidic channel 9 and into separation zone 10. The separation zone 10 comprises two separation columns 11 and 12 packed with a silicagel stationary phase, in this case Supelclean™, with an average particle size of 45 µm. The columns allow separation of a fraction comprising the thiophenic sulphur compounds from one or more fractions comprising other aromatic or aliphatic compounds and other non-thiophenic sulphur compounds. The heptane continues through microvalve 13, which is positioned to allow the heptane to enter channel 14 which leads to waste reservoir 15. The waste reservoir has a charcoal absorbent bed 16 to prevent solvent vapours escaping through vent 17. In this procedure, the volume of heptane used is 1.5 ml, and the time taken for introducing the heptane and conditioning the separation zone takes 5 minutes. Sample fluid from a filled syringe is fed through sample inlet 18 to microvalve 7 along channel 19. The microvalve 7 position, in addition to allowing heptane to pass from channel 8 to channel 9, also allows sample fluid to pass from channel 19 to channel 20, the latter of which leads to waste reservoir 15. The microvalve 7 is so designed so that the volume of sample fluid held therein is 2 µl.. The microvalve 7 comprises two valve channels (60 and 61, shown in Figure 6), which connect the different microfluidic channels, depending on the valve position, discussed further below in relation to Figure 6.

Valve 7 is switched so that the 2 µl of sample fluid within one of the microvalve channels is aligned with the heptane-filled channels 8 and 9. Further heptane (1ml) is then fed into the sample plate, which pushes the 2µl sample fluid through the separation zone 10. Excess reactant in the channels of valve 13 are flushed into channel 14 leading to waste reservoir 15. As the sample fluid is fed into the first separation zone 10, it is separated into a plurality of fractions. The elution time for the fraction comprising thiophenic components, together with other polyaromatic molecules, is based on retention time determined previously. However, a detector, such as a UV, NIR or MIR detector, could be used in order to determine when the thiophenic fraction elutes from the separation zone.

At the point when the relevant fraction of the diesel fuel sample elutes from the separation zone, and any unwanted fractions that precede the desired fraction have passed through valve 13 to waste channel 14, microvalve 13 is switched so that the heptane solvent and sample fraction flow passes through the reactant loop 23, and the combined mixture flows into channel 24 which leads to the reaction zone 25, and which contains the 10mg catalyst. The reaction zone is surrounded by resistively heated elements which are located in the base portion of the apparatus, and which fit into slots 26 in the sample plate adjacent to the reaction zone, and which maintain the temperature therein at about 75°C. The mixture in the reaction zone is held there for 5 minutes to form a product fluid comprising the sulphones. The reaction zone has a discharge channel 27 which leads to waste reservoir 15, which allows removal of excess heptane in the reaction zone, and also allows escape of vapours from the reaction zone.

Meanwhile, when the reaction mixture is being held within the reaction zone, a mixture of heptane and 1% methanol is pumped from reservoir 28 and into the meandering microfluidic channel 29. The meandering layout allows a relatively high pre-determined volume of the heptane/methanol second mixture, in this case 260 µl, to be stored accurately on the plate between the inlet 28 and a microvalve 30 in a space-efficient way.

Microvalve 30 at this point is positioned so as to allow the heptane/methanol mixture to proceed along channel 31 and into the second separation zone 32. The second separation zone 32 comprises a single column packed with a solid stationary phase of silicagel, having an average particle size of 45 µm, such as Supelclean™, which separates a fraction comprising the sulphone components of the product fluid from the reaction zone. The solid stationary phase is conditioned by the heptane/methanol solvent. The solvent then passes through the analysis zone (not shown), which in this case is a cell equipped with quartz windows suitable for UV/Visible optical analysis. A background spectrum is obtained at this point. The solvent continues through microvalve 33, and enters waste reservoir 15 via channel 34.

Microvalve 30 is then switched, and the pump that is used to feed heptane/methanol solvent from inlet 28 into microfluidic channel 29 is then reversed, so as to pull the reaction zone contents from the reaction zone, through microfluidic channel 35 and into the meandering microfluidic channel 29. Microvalve 30 then reverts to its previous position, and the pump pushes the reaction zone contents through channel 31 and into the second separation zone 32, where the product fluid resulting from the reaction zone is separated into a plurality of fractions, one of which comprises the sulphones. The fluid passes through the UV/Visible cell, where the sulphones can be detected. When the sulphone-containing fraction reaches microvalve 33, the valve switches so that the fraction can pass through channel 38 into a concentration zone 36, which is in the form of a generally cylindrically-shaped chamber with a tapered base. The concentration zone is surrounded by recesses or holes in the sample plate that accommodate resistively heated elements located in the base portion of the apparatus, similar to those used for heating the reaction zone. The concentration zone also has a vent 37 to allow vapours to escape. The purpose of the concentration zone is to allow the methanol/heptane solvent to evaporate, and hence to concentrate the sulphones present within the fraction of the product fluid fed thereto, the rate of which is increased by heating. The tapered base is advantageous, as a sample of the concentrated product fraction can be more easily removed with a syringe, inserted through vent 37, for further analysis if so desired, for example by GC or GCMS. The remaining product fractions are fed to the waste reservoir by appropriately varying the position of microvalve 33. The complete procedure takes 25 minutes. Channel 51 provides a passage between concentration zone 36 and waste reservoir 15.

The plates also comprise numerous screw or bolt holes, two of which 50 are identified in Figure 5.

It should be noted that different parts of the sample can be operated under different conditions of pressure. Thus, in this example, the sample plate up to the reactor, incorporating the first separation zone 10 and microvalve 13, is operated at pressure of 3 barg (0.4 MPa), whereas the portion of the sample plate incorporating the second separation zone is operated at a pressure of 3 to 10 barg (0.4 to 1.1 MPa). The concentration zone and waste reservoir are at atmospheric pressure.

In this example, the pumping means are piston pumps, and can take the form of filled syringes, the piston being driven by a syringe driver, or alternatively the sample plate itself can have chambers that act as the reservoir for the fluids, which chambers are adapted to interface with pistons from the base portion of the apparatus that push the fluids into the sample plate channels when they are actuated.

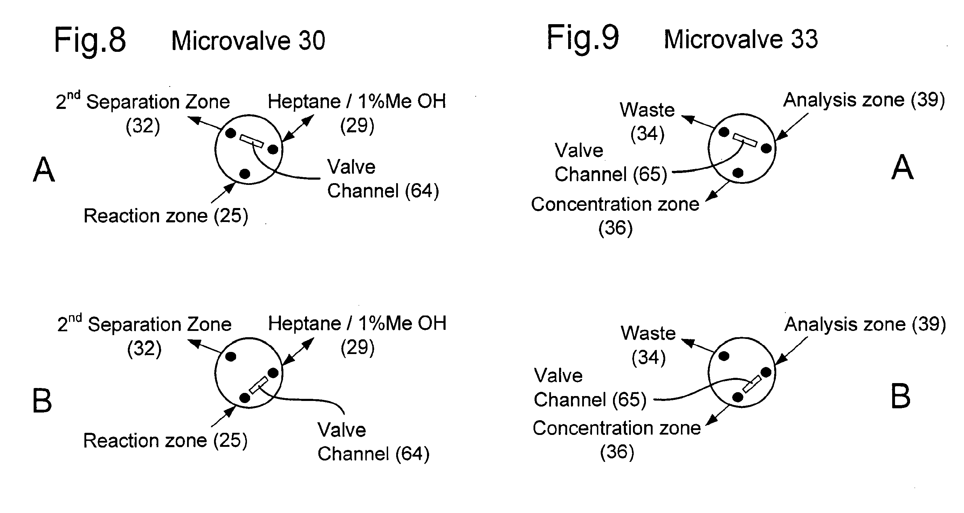

Figures 6 to 9 show further detail as to how the microvalves can direct the various fluids used in the analysis to the different regions or zones of the sample plate, by means of valve channels located therein. Figure 6 relates to microvalve 7, Figure 7 to microvalve 13, Figure 8 to microvalve 30 and Figure 9 to microvalve 33.

Figure 6 shows the positioning of two channels in microvalve 7 at different points in the analysis cycle.

Initially, the channel positions are set such that heptane is pumped from the heptane reservoir 5 into the first separation zone 10 via a valve channel 60 in microvalve 7, and sample fluid from sample inlet 18 is fed through a different valve channel 61. Valve channel 61 has a pre-specified volume, in this case 2 µl, and connects the channel 19 from the sample inlet 18 to channel 20, which leads to the waste reservoir 15. This configuration is shown by position A.

Once the 2 µl sample channel has been filled, and the separation zone 10 has been primed with heptane, the valve is rotated so that the sample-filled valve channel 61 is positioned between the heptane reservoir 5 and the first separation zone 10, enabling the sample to be pushed into the separation zone by delivery of fresh heptane from the heptane reservoir. This configuration is shown by position B.

Figure 7 shows the two channels in microvalve 13. The initial position of the two channels is such that hydrogen peroxide from reactant inlet 21 can be pumped into the 25µl reactant loop 23 through valve channel 63, and subsequently back through the valve through valve channel 62 and into microfluidic channel 14, which leads to the waste reservoir.

The microvalve is then rotated to allow heptane being pumped into and through the separation zone 10 to be fed directly to waste reservoir 15 via valve channel 63. This configuration is shown by position B. It should be noted that although there is a pathway from the reactant inlet 21 to the reactor 25 by means of valve channel 62, reactant is not being fed to the sample plate at this point.

When the desired fraction of the sample fluid elutes from the separation zone 10, the microvalve 13 is turned to position C, which allows the desired fraction to flow into reactant loop 23 through valve channel 62, and subsequently into reaction zone 25 through valve channel 63, taking with it the hydrogen peroxide present in the reactant loop.

Figure 8 shows the positions for the single valve channel 64 of microvalve 30. Position A shows the initial configuration, where methanol/heptane solvent is being fed from the inlet 28 and into the second separation zone 32. The microvalve is rotated to position B when the contents of the reaction zone 25 are to be sucked out by the same pump used to control the flow of methanol/heptane solvent through inlet 28, the reaction zone contents being used to fill the meandering microfluidic channel 29, and to ensure a volume of 260 µl is metered. Position A is re-adopted when the extracted reaction zone contents are fed to the second separation zone 32 and into the optical analysis zone.

Figure 9 shows the positions of the single valve channel 65 of microvalve 33. In position A, the valve channel is positioned so that fluid from second separation zone 32 and the analysis zone is directed to waste reservoir 15. In position B, the channel is configured so that the sulphone-containing fraction from the second separation zone 32 and from the analysis zone is fed to concentration zone 36.

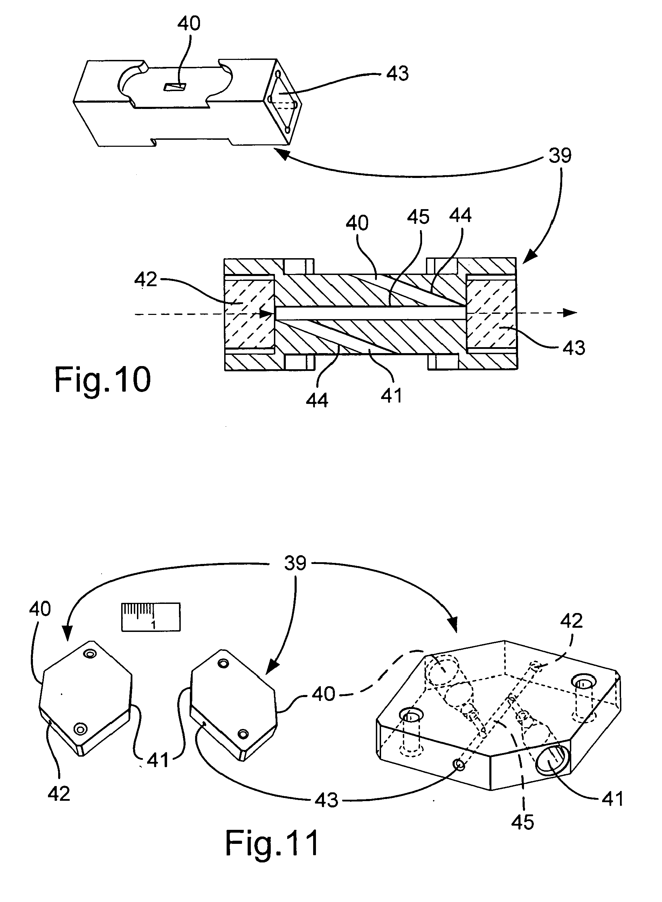

Two different examples of UV/Visible cells 39 suitable for use with the sample plate and base portion are shown in Figures 10 and 11. The UV/Visible cell is a separate apparatus whose inlet 40 and outlet 41 are suitably adapted to provide a leak-free seal with the relevant microfluidic channels of the sample plate. Additionally, the cell is also accommodated appropriately by the base portion of the apparatus, such that the quartz windows 42 and 43 align with the emitted UV/Visible frequencies by the optical analysis device also in the base portion of the apparatus, which are directed into the cell through window 42 and which are transmitted from the cell through window 43, which transmitted radiation is then directed to a suitable UV/Visible detector. The fluid to be analysed flows through microfluidic channels 44 within the UVNisible cell, at least a portion of which 45 defines the absorption pathlength. In Figure 11, holes in the housing of the cell are shown which engage with struts in the base portion to ensure the cell is positioned appropriately so that EMR can be directed from an emitter into the cell, and from the cell to an EMR detector. In Figure 8, the indented shape of the cell serves the same purpose.

The cell is designed to ensure that the absorption pathlength is sufficiently large to allow quantitative and qualitative analysis to be performed, while being small enough to ensure that the cell is compact and does not impact too greatly on the overall size of the portable apparatus. Additionally, in order to minimise disturbance of fluids flowing through the cell, a Z-shaped channel conformation can be used, as described in EP-A-0 266 769.

The cell can be re-used, and also washed separately to the sample plate and base portion where necessary. This is advantageous if the sample plate is disposable, and where the base portion comprises complex and sensitive electronics and other components which could suffer damage through washing.

It should be noted that a UV/Visible cell like this can be readily adapted to allow different fluids to be analysed, and for different types of optical analysis to be performed, for example through suitable choice of inert cell materials, and by employing windows that are sufficiently transparent to the EMR used for the analysis.

## Claims

1. A sample plate for a portable analysis apparatus for the analysis of a fluid, which sample plate comprises a sample inlet, a reaction zone, an analysis zone, and at least one separation zone, the sample plate being adapted to allow;
(a) a sample fluid to be fed to the sample plate, through the inlet to the reaction zone or optionally to a separation zone, which separation zone separates the sample fluid into two or more fractions at least one of which is fed to the reaction zone;
(b) a reactant to be fed to the reaction zone;
(c) the reaction zone to be maintained under conditions that enable reaction to occur between the reactant and the sample fluid or fraction thereof to produce a product fluid;
(d) transfer of the product fluid to the analysis zone or optionally to a separation zone in which the product fluid is separated into two or more fractions, at least one of which is transferred to the analysis zone.

2. A sample plate as claimed in claim 1, in which the separation zones of paragraph (a) and of paragraph (d) are both present.

3. A sample plate as claimed in claim 1 or claim 2, having microfluidic channels for directing fluids to the relevant zones of the sample plate.

4. A sample plate as claimed in claim 3, having one or more microvalves for controlling fluid pathways.

5. A sample plate as claimed in any one of claims 1 to 4, in which one or more of the separation zones comprises a solid stationary phase for separating the sample fluid and/or the product fluid into two or more fractions.

6. A sample plate as claimed in any one of claims 1 to 5, additionally comprising a concentration zone.

7. A sample plate as claimed in any one of claims 1 to 6, in which the concentration zone has a tapered base.

8. A sample plate as claimed in any one of claims 1 to 7, having a reservoir for waste.

9. A sample plate as claimed in any one of claims 1 to 8, having one or more vents.

10. A sample plate as claimed in claim 9, in which one or more of the vents have an absorbent.

11. A sample plate as claimed in any one of claims 1 to 11, in which the analysis zone is suitable for performing optical analysis.

12. A sample plate as claimed in claim 11, in which the analysis zone has one or more windows that are transparent to the electromagnetic radiation to be used in the optical analysis.

13. A sample plate as claimed in any one of claims 1 to 12, in which the analysis zone can be detached from the sample plate.

14. A sample plate as claimed in any one of claims 1 to 13, having one or more reservoirs for storage of one or more fluids that are used or created during the analysis.

15. A sample plate as claimed in any one of claims 1 to 14, in which a sample fluid, a solvent and a catalyst can all be fed to the reaction zone, and a second solvent can be fed to a separation zone situated downstream of the reactor.

16. A portable apparatus for the analysis of a fluid sample comprising a sample plate and a base portion, **characterised by** the sample plate being in accordance with any one of claims 1 to 15.

17. A portable apparatus as claimed in claim 16, in which the temperature of different regions of the sample plate can be heated or cooled.

18. A portable apparatus as claimed in claim 17, in which the base portion comprises heating elements, and the sample plate is adapted to accommodate the heating elements.

19. A portable apparatus as claimed in claim 18, in which the sample plate comprises a concentration zone, and the reaction zone and concentration zone are adapted to be heated by heating elements located in the base portion of the apparatus.

20. A portable apparatus as claimed in any one of claims 16 to 19, in which the analysis zone of the sample plate is suitable for optical analysis, and the base portion comprises an emitter and a detector of the electromagnetic radiation to be used.

21. A portable apparatus as claimed in any one of claims 16 to 20, in which the sample plate has one or more microvalves, and the base portion of the apparatus comprises one or more pumps.

22. A portable apparatus as claimed in claim 21, in which the one or more pumps are selected from diaphragm pumps, peristaltic pumps, rotary pumps, gear pumps and piston pumps.

23. A portable apparatus as claimed in claim 21 or claim 22, in which the base portion comprises actuators for any microvalves and/or pumps.
